# EUROPEAN PATENT APPLICATION

(11) **EP 4 691 341 A1**
(43) Date of publication of application: **11.02.2026**
(21) Application number: 23930833.1
(22) Date of filing: 16.11.2023
(51) Int. Cl.: A61B 3/028, A61B 3/113, G02C 7/06, G02C 13/00

(54) **METHOD FOR MEASURING BEHAVIOR PATTERN UPON VISUAL LINE MOVEMENT, METHOD FOR DETERMINING PROGRESSIVE REFRACTION LENS, AND DEVICE FOR MEASURING BEHAVIOR PATTERN UPON VISUAL LINE MOVEMENT**

(30) Priority: 31.03.2023 JP 2023057358
(71) Applicant: Hoya Lens Thailand Ltd., Pathumthani 12130 (TH)
(72) Inventor: ITO Ayumu, Tokyo 160-8347 (JP); SONEHARA Toshiaki, Tokyo 160-8347 (JP)
(74) Representative: Betten & Resch
(86) International application number: PCT/JP2023/041190
(87) International publication number: WO 2024/202206

(57) **Abstract**

There is provided a method for measuring a behavioral pattern during gaze shifting, the method including: displaying an optotype on at least one of multiple display units arranged at different distances from a subject wearing a progressive power lens, and allowing the subject to view the optotype; and obtaining a subjective reaction from the subject who views the optotype, wherein by repeating the viewing the optotype and the obtaining the subjective reaction for multiple optotypes, the subject is allowed to experience two or more different viewing states, and a change in subject's behavior between the viewing states are measured.

## Description

### Technical Field

The present invention relates to a method for measuring a behavioral pattern during gaze shifting, a method for determining a progressive power lens, and a device for measuring a behavioral pattern during gaze shifting.

### Description of related art

Various methods have been proposed for designing a progressive power lens suited to a subject. For example, patent document 1 discloses a method, which is a method for determining a person's visual behavior, the method including: recording a movement of a person's head while performing a visual task; recording at least one eyeball movement of the person while performing a visual task; determining a relative orientation of the eyeballs with respect to the head at different times; and determining an amount of time required for keeping the eyeballs in each orientation.

Also, patent document 2 discloses a method for evaluating an ophthalmic lens, the method including: a task setting step in which an ophthalmic lens suitability testing device displays a plurality of optotypes at a plurality of different display positions, sets a predetermined task to a subject wearing the ophthalmic lens, and allows the subject to answer the task for each of the plurality of optotypes; a task evaluation step in which the ophthalmic lens suitability testing device determines an evaluation score for the task at each of the plurality of display positions using correctness or incorrectness of the answers to the task performed based on the results; and a lens evaluation step in which the ophthalmic lens suitability testing device evaluates suitability of the ophthalmic lens for the subject, using the evaluation score of the task.

### Prior art document

### Patent document

[Patent Document 1] Patent No. 4659027
[Patent Document 2] Patent No. 6405662

### Summary of the invention

### Problem to be solved by the invention

An object of one embodiment of the present invention is to provide a technique for measuring a behavioral pattern of a subject during gaze shifting, which provides useful information for determining a progressive power lens better suited to the subject.

### Means for solving the problem

A first aspect of the present invention is a method for measuring a behavioral pattern during gaze shifting, the method including: displaying an optotype on at least one of multiple display units arranged at different distances from a subject wearing a progressive addition lens, and allowing the subject to view the optotype; and obtaining a subjective reaction from the subject who views the optotype, wherein by repeating the viewing a plurality of optotypes and obtaining the subjective reaction, the subject is allowed to experience two or more different viewing states and his or her behavior is measured between the different viewing states.

A second aspect of the present invention is the method for measuring a behavioral pattern during gaze shifting according to the first aspect, wherein the change in behavior includes a change in at least one of the following: whether the subjective reaction is correct or incorrect; the reaction time from when the optotype is displayed until the subjective reaction is obtained; and a movement of a subject's head or eyeball from when the optotype is displayed until the subjective reaction is obtained.

A third aspect of the present invention is the method for measuring a behavioral pattern during gaze shifting according to the first aspect, wherein the two or more different viewing states include a near vision state and a far vision state.

A fourth aspect of the present invention is the method for measuring a behavioral pattern during gaze shifting according to the first aspect, wherein when repeating the viewing the optotype and the obtaining the subjective reaction, the optotype is displayed in a certain order so that the subject performs a series of gaze shifting at a discrete viewing distance.

A fifth aspect of the present invention is the method for measuring a behavioral pattern during gaze shifting according to the first aspect, wherein in the viewing the optotype and the obtaining the subjective reaction, the subject is continuously photographed, and a movement of a subject's head or eyeballs is measured from when the optotype is displayed until when the subjective reaction is obtained.

A sixth aspect of the present invention is a method for determining a progressive power lens, the method further including:
displaying an optotype on at least one of multiple display units arranged at different distances from a subject wearing a progressive power lens, and allowing the subject to view the optotype; and
obtaining a subjective reaction from the subject who views the optotype,
the method further comprising:
   allowing the subject to experience two or more different viewing states by repeating the viewing of the optotype and the obtaining the subjective reaction for multiple optotypes, and measuring a change in subject's behavior between the viewing states; and
   selecting a design of the progressive power lens suited to the subject based on data on the change in behavior.

A seventh aspect of the present invention is a device for measuring a behavioral pattern during gaze shifting, the device including:
multiple display units arranged at different distances from a subject wearing a progressive power lens, for displaying optotypes to be viewed by the subject;
an input unit into which the subject who views the optotype inputs a subjective reaction; and
a measurement unit that allows the subject to experience two or more different viewing states by repeating the display of the optotypes and the input of the subjective reaction, for measuring a change in subject's behavior between the viewing states.

An eighth aspect of the present invention is the device for measuring a behavioral pattern during gaze shifting according to the seventh aspect, the device further including an imaging unit that continuously images the subject.

### Advantage of the invention

According to one embodiment of the present invention, a behavioral pattern of a subject during gaze shifting can be measured, which provides useful information for determining a progressive power lens better suited to the subject.

### Brief description of the drawings

[FIG. 1] FIG. 1 is a flowchart showing an example of a method for measuring a behavioral pattern during gaze shifting and a method for determining a progressive power lens, according to a first embodiment of the present invention.
[FIG. 2] FIG. 2 is a schematic view for explaining a gaze shifting task, according to the first embodiment of the present invention.
[FIG. 3] FIG. 3 (a) and 3 (b) are schematic views showing an example of a display unit that displays optotypes, according to the first embodiment of the present invention.
[FIG. 4] FIG. 4 is a schematic view for explaining a display position of an optotype 30 on the display unit, according to the first embodiment of the present invention.
[FIG. 5] FIG. 5 is a view for plotting data on a behavioral pattern (reaction time and postural stability) measured during gaze shifting of each subject, according to an example of the present invention.
[FIG. 6] FIG. 6 is a view for plotting data on a behavioral pattern (asymmetry of reaction time) measured during gaze shifting of each subject, according to an example of the present invention.

### Detailed description of the invention

### <Finding obtained by the inventors>

First, finding obtained by the inventors will be described. As progress is made in individually designing a progressive power lens suited to each subject, methods have been proposed to obtain information such as a relationship between the subject's eyeball movement and head movement and a change in visual distance while the subject is performing a specific task (for example, reading or using a tablet), and to reflect this information in an optical design of the progressive power lens. The area in the progressive power lens that is used when performing such a specific task can be said to be a relatively limited area with respect to an entire lens, when considering the gaze shifting and the change in visual distance.

In contrast, it is found that a most common situation in which the subject feels dissatisfied with a progressive power lens (e.g. has difficulty in focusing) is when the subject looks up to look at a distant object or when the subject looks away and then back again (for example, in real life, when reading, watching television, and then going back to reading). In order to reproduce a situation of switching between viewing (focusing) objects at different distances and measure the behavioral pattern of the subject during gaze shifting, it is required to change the viewing distance and a display position of the optotype more dynamically and to significantly change the area of use of the progressive power lens, instead of shifting the gaze to follow an object (quasi-static visual behavior). Simultaneously, in consideration of a common feature of changing the viewing distance once and then returning to the same viewing distance, it is also important to allow the subject to experience a series of movements in which he or she focuses the gaze on a certain visual object (hereafter referred to as a viewing state), ends that viewing state by moving their eyes away or toward a different object, and then resumes the same viewing state, in order to reproduce a wearing state of the progressive power lens in real life.

The inventors have conducted extensive research into the above-described problem. As a result, for example, there is provided a method for measuring a behavioral pattern during gaze shifting, the method including: displaying optotypes on at least one of multiple display units arranged at different distances from a subject wearing a progressive power lens, and allowing the subject to view the optotypes; and obtaining a subjective reaction from the subject who views the optotypes, wherein by repeating the viewing the plurality of optotypes and obtaining the subjective reaction, the subject is allowed to experience two or more different viewing states and his or her behavior is measured between the different viewing states. At this time, when changing to a different viewing state, it can be considered that characteristics of the behavioral pattern will appear in the directivity of the change, so it is also useful to measure the change in the opposite direction.

Specifically, by using different lens areas, such as distance and near portions of the progressive power lens, the subject is allowed to repeatedly experience the change in a viewing state, then, the accuracy of reading the optotype after change of the viewing state, the change in reaction time, and the change in the subject's head or eyeball movements are measured, to quantify a refocusing ability and proficiency in using the progressive power lens, of the subject wearing the progressive power lens. It is also possible to qualitatively capture the change in a subject's behavior between viewing states and classify it as an attribute of the individual subject. Data on the behavioral pattern of the subject during gaze shifting measured in this manner provides useful information for determining a progressive power lens better suited to the subject.

### [Details of the embodiment of the present invention]

Next, an embodiment of the present invention will be described below with reference to the drawings. The present invention is not limited to these examples, but is defined by the claims, and is intended to include all modifications within the meaning and scope of the claims.

### <First embodiment of the present invention> (1) Method for measuring a behavioral pattern during gaze shifting and method for determining a progressive power lens

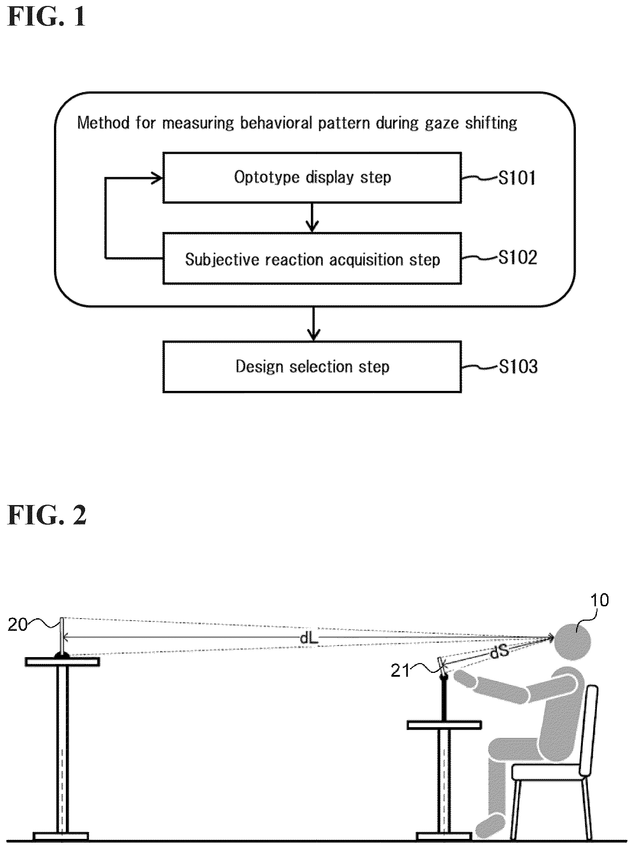

First, a method for measuring a behavioral pattern during gaze shifting and a method for determining a progressive power lens according to this embodiment will be described. FIG. 1 is a flowchart showing an example of a method for measuring a behavioral pattern during gaze shifting and a method for determining a progressive power lens, according to a first embodiment of the present invention. As shown in FIG. 1, the method for measuring a behavioral pattern during gaze shifting according to the this embodiment includes, for example, an optotype display step S101 and a subjective reaction acquisition step S102, and by repeating the above two steps multiple times, the subject is allowed to experience two or more different viewing states, and the change in subject's behavior between the viewing states is measured multiple times. Hereinafter, the task of repeating the optotype display step S101 and the subjective reaction acquisition step S102 multiple times is referred to as a "gaze shifting task" in this specification. When the present invention is applied as a method for determining a progressive power lens, a design selection step S103 may be performed after measuring the subject's behavioral pattern during gaze shifting by a gaze shifting task.

FIG. 2 is a schematic view for explaining a gaze shifting task, according to the first embodiment of the present invention. As shown in FIG. 2, multiple display units (in this embodiment, a first display unit 20 and a second display unit 21) are arranged at different distances from a subject 10 wearing the progressive power lens. The first display unit 20 is, for example, a display terminal such as a tablet, and is a display unit for displaying an optotype assuming a distant vision state (a state in which the distance portion of the progressive power lens is being used). The first display unit 20 is arranged, for example, such that front gaze for far vision of the subject 10 (for example, visual distance dL=3 m) is at the center of a screen. The second display unit 21 is, for example, a display terminal such as a smartphone, and is a display unit for displaying an optotype assuming a near vision state (a state in which the near portion of the progressive power lens is being used). The second display unit 21 is arranged, for example, so that downward gaze for near vision (e.g., visual distance dS = 40 cm, downward 30 degrees) of the subject 10 is at the center of the screen.

It is preferable that the difference in visual distance dM (=dL- dS) between the first display unit 20 and the second display unit 21 is beyond a range in which, when the subject 10 exerts a certain degree of focus adjustment on the visual object, the subject feels that the object is in focus even when the position of the visual object does not coincide with a focus adjustment position. Alternatively, it is preferable that the range be beyond a range where blur is not noticeable (depth of field). That is, it is preferable that the multiple displays used in the gaze shifting task are arranged at different distances that are greater than or equal to the depth of field. Here, the depth of field depends on the influence of aberration, pupil diameter, and the visual angle of the visual object, but according to a model eye calculation ignoring aberration, the depth of field of an eyeball with a pupil diameter of 4 mm and decimal visual acuity of 1.0 is approximately 0.15 D (See Optometry Expert: Optics and Glasses (Igaku-Shoin, 2018)). This makes it impossible for the subject 10 to focus on multiple display units (in this embodiment, the first display unit 20 and the second display unit 21) at the same time, and therefore more dynamic gaze shifting is required, making it easier to measure the behavioral pattern during gaze shifting.

### (Optotype display step S101, subjective reaction acquisition step S102)

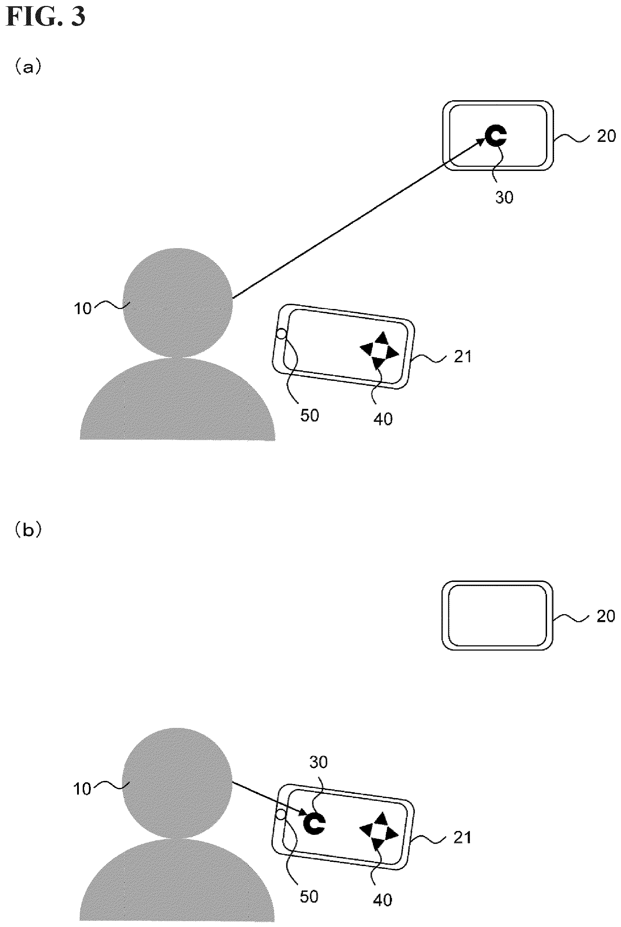

The optotype display step S101 is a step of displaying optotypes on at least one (in this embodiment, any one) of multiple display units (in this embodiment, the first display unit 20 and the second display unit 21) and allowing the subject 10 to view the optotypes. FIG. 3 (a) and 3 (b) are schematic views showing an example of a display unit that displays an optotype, according to the first embodiment of the present invention. FIG. 3 (a) shows an example in which the optotype 30 is displayed on the first display unit 20 and the optotype 30 is not displayed on the second display unit 21. FIG. 3 (b) shows an example in which the optotype 30 is displayed on the second display unit 21 and the optotype 30 is not displayed on the first display unit 20. In FIGS. 3 (a) and 3 (b), the arrow pointing out from the subject 10 indicates the gaze of the subject 10. As shown in FIGS. 3(a) and 3(b), in the optotype display step S101 of this embodiment, the optotype 30 is displayed on only one of the display units, making it easy for the subject 10 to recognize the optotype 30 that he or she should view. Further, by changing the display unit on which the optotype 30 is displayed, the subject 10 is allowed to experience different viewing states. The optotype 30 displayed on the display unit is not particularly limited as long as it can be clearly viewed when the subject 10 focuses on it. In this embodiment, as shown in FIG. 3 (a) and FIG. 3 (b), a Landolt ring (equivalent to a visual acuity of 0.7) is used as the optotype 30.

The subjective reaction acquisition step S102 is a step of obtaining a subjective reaction from the subject 10 viewing the optotype 30 displayed in the optotype display step S101. In this embodiment, the subject 10 uses an input unit 40 to answer the direction of a break of the Landolt ring. As shown in FIG. 3 (a) and FIG. 3 (b), the input unit 40 is, for example, software keys displayed on a smartphone or the like, and in this embodiment, the second display unit 21 also serves as the input unit 40.

When the subjective reaction of the subject 10 is obtained in the subjective reaction acquisition step S102, the optotype display step S101 is performed again. Specifically, for example, when the optotype 30 is displayed on the first display unit 20 in the previous optotype display step S101, the optotype 30 on the first display unit 20 is erased and the optotype 30 is displayed on the second display unit 21, as shown in FIG. 3 (b). Alternatively, when the optotype 30 is displayed on the second display unit 21 in the previous optotype display step S101, the optotype 30 on the second display unit 21 is erased and the optotype 30 is displayed on the first display unit 20, as shown in FIG. 3 (a). In this way, in the optotype display step S101, it is preferable to allow the subject 10 to alternate between viewing the optotype 30 displayed on the first display unit 20 and the optotype 30 displayed on the second display unit 21 (i.e., alternate between near vision and far vision). This allows the subject 10 to shift his or her gaze at discrete viewing distances, such as from far vision to near vision or from near vision to far vision, and therefore more dynamic gaze shifting is allowed, making it easier to measure the behavioral pattern during gaze shifting.

As described above, the optotype display step S101 and the subjective reaction acquisition step S102 are repeated multiple times to allow the subject 10 to experience two or more different viewing states (in this embodiment, a near vision state and a far vision state), and the change in the behavior of the subject 10 between the viewing states is measured multiple times, to thereby measure the behavioral pattern of the subject 10 during gaze shifting. The change in the behavior of the subject 10 include, for example, the change in at least any of the following: whether the subjective reaction of the subject 10 is correct or incorrect, the reaction time from when the optotype 30 is displayed until the subject obtains the subjective reaction, and the movement of the head or eyeballs of the subject 10 from when the optotype 30 is displayed until the subject obtains the subjective reaction, and preferably include all of these changes. Specifically, for example, when the subject 10 exhibits a behavioral pattern during gaze shifting that results in many incorrect subjective reactions, it may be determined that the subject 10 has a low ability to refocus or does not have much proficiency in using the progressive power lens, and for example, when there is a large difference in the reaction time when shifting the gaze from near vision to far vision and from far vision to near vision, it may be determined that there is asymmetry in the reaction time of the subject 10, and for example, when there are a lot of unnecessary head and eyeball movements as a behavioral pattern during gaze shifting, it may be determined that the subject 10 has low postural stability. These behavioral patterns during gaze shifting suggest that a design of the progressive power lens and a wearing manner are not as intended by a designer. On the other hand, for example, when the behavioral pattern during gaze shifting is smooth (i.e., the posture is stable), the reaction time is short, and there is no subjective incorrect reaction, it may be determined that the subject 10 has good refocusing ability and proficiency in using the progressive power lens. At this time, it can be assumed that the behavioral pattern during gaze shifting is desirable and intended by a designer. By measuring the above-described multiple behavior changes (parameters) multiple times, the behavioral pattern during gaze shifting can be measured from a more multifaceted perspective.

In the optotype display step S101 and the subjective reaction acquisition step S102, it is preferable to continuously capture images of the face of the subject 10 and measure the movement of the head or eyeballs of the subject 10 from when the optotype 30 is displayed until the subjective reaction is obtained. In this embodiment, as shown in FIGS. 3 (a) and 3 (b), the face of the subject 10 is continuously captured by an imaging unit 50 (e.g., a camera of a smartphone) provided in the second display unit 21. That is, in this embodiment, the second display unit 21 also serves as the imaging unit 50. By applying a known technique such as image recognition to the image captured by the imaging unit 50, data on the head movement, gaze shifting, interpupillary distance, etc., of the subject 10 can be obtained, allowing to measure the behavioral pattern during gaze shifting from a more multifaceted perspective.

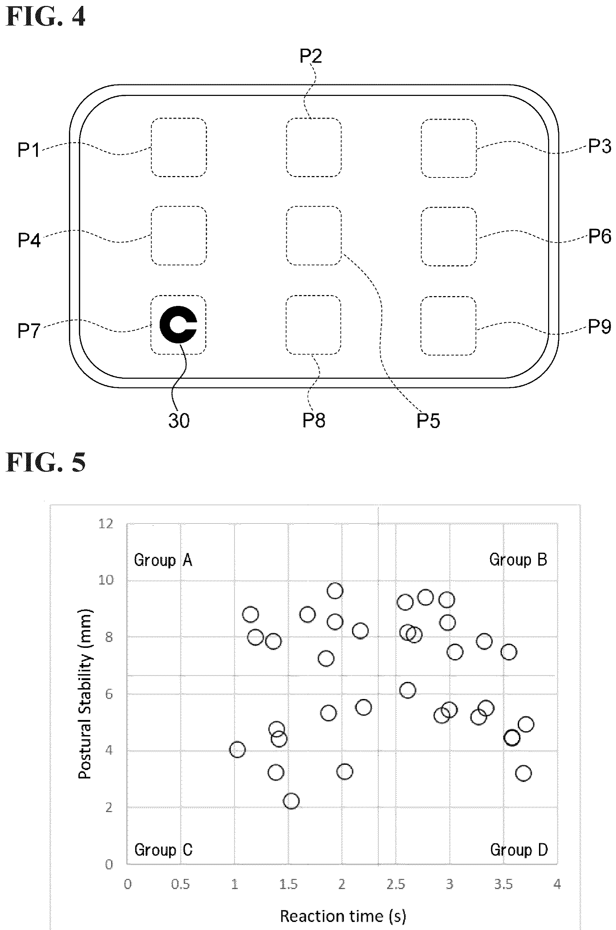

FIG. 4 is a schematic view for explaining a display position of an optotype 30 on the display unit. In FIG. 4, the optotype 30 is displayed at a display position P7 (lower left of the screen), but in the optotype display step S101, it is preferable to randomly display the optotype 30 (for example, at any of display positions P1 to P9) within the screen of the display unit. When the display position of the optotype 30 is the same every time (for example, the display position P5 in the center of the screen every time), it is probable that the subject 10 predicts the display position of the optotype 30 and shifts his or her gaze before the optotype 30 is displayed, making it difficult to accurately measure the behavioral pattern during gaze shifting. In contrast, by randomly displaying the optotype 30, it becomes difficult for the subject 10 to predict the display position of the optotype 30, and therefore it becomes easier to accurately measure the behavioral pattern during gaze shifting. It is preferable that the display position of the optotype 30 within the screen of the display unit is changed so that the visual distance remains almost unchanged (for example, the change is less than the depth of field of 0.15 to 0.2 D).

By performing the above-described gaze shifting task, the behavioral pattern of the subject 10 during gaze shifting can be measured, which is useful information for determining a progressive power lens that is more suited to the subject 10.

### (Design selection step S103)

The design selection step S103 is a step of selecting a design of the progressive power lens suited to the subject 10 based on data on the behavioral pattern during gaze shifting (data on the change in the behavior of the subject 10) measured, for example, by the gaze shifting task. Specifically, for example, data on the behavioral pattern during gaze shifting when wearing the progressive power lens of design A is compared with data on the behavioral pattern during gaze shifting when wearing the progressive power lens of design B, and a design may be selected which is determined to be more desirable in terms of the behavioral pattern during gaze shifting as intended by a designer, and for example, for the subject 10 whose behavioral pattern during gaze shifting is not as intended by a designer, an improved design may be selected that is easier to refocus, with a trade-off with a quality (image quality) on a principal meridian, and for the subject 10 whose behavioral pattern during gaze shifting is as intended by a designer, a design that emphasizes quality on the principal meridian may be selected. In the design selection step S103, for example, a lens having a design suited to the subject may be selected from a plurality of customized lenses prepared in advance.

By applying the above-described method to a plurality of subjects, a design of the progressive power lens suited to the needs of each individual subject can be provided.

### (2) Device for measuring a behavioral pattern during gaze shifting

The present invention can also be applied as a device for measuring the behavioral pattern of the subject 10 during gaze shifting. The device for measuring a behavioral pattern during gaze shifting according to this embodiment includes for example: multiple display units (e.g., the first display unit 20 and the second display unit 21) arranged at different distances from the subject 10 wearing a progressive power lens, for displaying the optotype 30 to be viewed by the subject 10; the input unit 40 into which the subjective reaction of the subject 10 viewing the optotype 30 is inputted; and a measurement unit that allows the subject 10 to experience two or more different viewing states (e.g., near vision and far vision) by repeating the display of the visual target 30 and the input of the subjective reaction multiple times, and measures the change in the behavior of the subject 10 between the viewing states multiple times. The measurement unit is, for example, an information processing device capable of processing and storing data, and the first display unit 20 or the second display unit 21 may also function as the measurement unit. In addition, it is preferable that the device for measuring the behavioral pattern during gaze shifting further includes an imaging unit 50 for continuously capturing images of the face of the subject 10.

### <Other embodiments of the present invention>

Although the embodiment of the present invention has been specifically described above, the present invention is not limited to the above embodiment, and various modifications can be made without departing from the gist of the present invention.

For example, the above embodiment shows a case in which the first display unit 20 and the second display unit 21 are arranged as the multiple display units used in the gaze shifting task, but three or more display units may be arranged. Further, the display unit may be arranged at a position such as the side of the subject 10, that is, at a position other than the front. Further, it is not required to use multiple display devices in order to arrange multiple display units, and for example, a portion of one display device may be used as the first display unit 20 and another portion may be used as the second display unit 21.

Further, for example, as shown in FIG. 2, the above embodiment shows a case in which the first display unit 20 is arranged far in front of the subject 10 and the second display unit 21 is arranged near downward (30 degrees downward) the subject 10. However, the angle (viewing angle) at which the multiple display units are arranged is not limited to the above embodiment. However, it is preferable that the multiple display units are arranged so that they do not overlap when viewed from the subject 10 and so that the viewing angles from the subject 10 are different. Further, from the viewpoint of making it easier for the subject 10 to view the optotype 30 in a natural posture, as described in the above embodiment, it is preferable that the display unit corresponding to far vision (first display unit 20) is arranged in front of the subject 10, and the display unit corresponding to near vision (second display unit 21) is arranged downward the subject 10.

Further, for example, the above embodiment shows a case in which a Landolt ring equivalent to a visual acuity of 0.7 is used as the optotype 30, but the form of the optotype 30 is not limited to the above embodiment. Specifically, for example, arrows and letters may be used, or simple shapes (circles and polygons) may also be used. It is preferable that the size of the optotype 30 is adjusted according to a distance so that a visual angle does not vary significantly depending on a viewing distance. From the viewpoint of effectively measuring the behavioral pattern during gaze shifting, which is an object of the present invention, it is preferable that the size be such that it can only be viewed firmly from the front, which is a size equivalent to a visual acuity of about 0.7 to 1.0.

Further, for example, the above embodiment shows a case in which the second display unit 21 is used as the input unit 40, and the subjective reaction from the subject 10 is obtained using software keys displayed on the second display unit 21. However, the form of the input unit 40 is not limited to the above embodiment. For example, the input unit 40 may be an input device equipped with a lever or a cross key, or a voice input device that obtains the subjective reaction from the subject 10 through vocalization, or may be configured so that the subjective reaction of the subject 10 can be manually input by a measurement assistant. From the viewpoint of preventing the subject 10 from shifting his or her gaze to anything other than the optotype 30, it is preferable that the subjective reaction can be inputted into the input unit 40 while the subject 10 keeps his or her gaze fixed on the optotype 30.

Further, for example, the above embodiment shows a case in which the movement of the head or eyeballs of the subject 10 is measured from when the optotype 30 is displayed until the subjective reaction is obtained, by using a camera provided in the second display unit 21 as the imaging unit 50 to continuously capture images of the face of the subject 10. However, the manner of measuring the movement of the head or eyeballs of the subject 10 is not limited to the above embodiment. for example, the distance between the second display unit 21 and the head of the subject 10 and the interpupillary distance of the subject 10 may be measured using a LIDAR (Laser Imaging Detection and Ranging) provided in a smartphone or the like.

Further, for example, the above embodiment shows a case in which the subject 10 is allowed to alternately view the optotype 30 displayed on the first display unit 20 and the optotype 30 displayed on the second display unit 21 (i.e., the case in which the subject 10 is allowed to alternate between near vision and far vision). However, in the optotype display step S101, it is not always required to alternate between near vision and far vision every time. For example, during the optotype display step S101, which is performed multiple times, the subject 10 rarely shifts his or her gaze from near vision to near vision or from far vision to far vision, making it difficult for the subject 10 to predict the display position of the optotype 30, and thus making it easier to accurately measure the behavioral pattern during gaze shifting.

Further, for example, the above embodiment shows a case in which in the optotype display step S101, the optotype 30 is displayed only on one of the multiple display units (the first display unit 20 and the second display unit 21). However, in the optotype display step S101, it is sufficient that the optotype 30 is displayed on at least one of the multiple display units, and the optotype 30 may be displayed on the multiple display units simultaneously. Specifically, for example, the optotype 30 may be displayed on both the first display unit 20 and the second display unit 21, and the subject 10 may be allowed to view the optotype 30 displayed at a brightly lit position, or the subject 10 may be allowed to view the optotype 30 displayed at a position pointed to by a pointer or the like. Even in such a case, the subject 10 is allowed to experience two or more different viewing states, enabling to measure the behavioral pattern during gaze shifting.

### Examples

Next, examples of the present invention will be described. These examples are merely examples of the present invention, and the present invention is not limited to these examples.

In this example, 34 subjects took a gaze shifting task under the following conditions. As shown in FIG. 2, the multiple display units were arranged such that the first display unit 20 (tablet) was arranged in front of the subject 10 (visual distance dL = 3 m) and the second display unit 21 (smartphone) was arranged 30 degrees downward the subject 10 (visual distance dS = 40 cm). The used optotype 30 was a Landolt ring equivalent to a visual acuity of 0.7, and the optotype 30 was alternately displayed on the first display unit 20 and the second display unit 21, 30 times in total, and the subject 10 was asked to answer the direction of the break of the Landolt ring. The answer (subjective reaction) was given using the software keys (input unit 40) displayed on the second display unit 21. While the gaze shifting task was being performed, images were captured continuously (30 times per second) using a camera (imaging unit 50) provided in the second display unit 21, and the movement of the head of the subject 10 (specifically, the change over time in the distance between the second display unit 21 and the face of the subject 10) was measured using image recognition.

### (1) Reaction time and postural stability

Data on the behavioral pattern (reaction time and postural stability) of each subject during gaze shifting measured using the above gaze shifting task is plotted and organized in FIG. 5. In FIG. 5, the horizontal axis indicates the average value of the reaction time when switching from far vision to near vision (average reaction time), and the vertical axis indicates the postural stability. In this example, the postural stability is an index of the subject's large postural change synchronized with the answer, obtained by extracting low-frequency components (10 Hz or less) from the change in the distance between the second display unit and the subject's face over time and then taking the root mean square (RMS) of the fluctuation range. A smaller value of the postural stability indicates a more stable posture.

In the results shown in FIG. 5, the average reaction time across all subjects was 2.4 seconds, and the average postural stability across all subjects was 6.5 mm. Therefore, the group of the subjects with an average reaction time of less than 2.4 seconds and a postural stability of 6.5 mm or more was designated group A (upper left of FIG. 5), and the group of the subjects with an average reaction time of 2.4 seconds or more and a postural stability of 6.5 mm or more was designated group B (upper right of FIG. 5) and the group of the subjects with an average reaction time of less than 2.4 seconds and a postural stability of less than 6.5 mm was designated group C (lower left of FIG. 5 ), and the group of the subjects with an average reaction time of 2.4 seconds or more and a postural stability of less than 6.5 mm was designated group D (lower right of FIG. 5 ). That is, the subjects were classified as follows: group A, with fast reaction but unstable posture; group B, with slow reaction and unstable posture; group C, with fast reaction and stable posture; and group D, with slow reaction but stable posture.

The subjects in group C were regarded as having a high level of proficiency in using the progressive power lens, and for the group C, a design of the progressive power lens that the subject was currently wearing or a design that emphasized quality on the principal meridian, was selected. The subjects in group B were regarded as having a low level of proficiency in using the progressive power lens, and for the group B, a lens design for initial wear or a design with a short progressive zone length that would reduce gaze shifting, was selected. For example, it is also possible to give a lecture to the subjects in group B regarding a manner of gazing while wearing the progressive power lens. In addition, for the subjects in group D, since they showed characteristics of little posture deviation when switching to near vision and a long reaction time, a design (e.g., a hard design) was selected that allowed distortion in the intermediate portion, with the clear vision area in the near portion widened.

In this example, the measured data on the behavioral pattern during gaze shifting was used to classify the subjects. For example, by allowing the same subject to wear multiple progressive power lenses and comparing data on the behavioral patterns during gaze shifting for each lens, which design of the progressive power lens is more suitable can be determined. Specifically, for example, by comparing designs with long and short progressive zone lengths, hard and soft designs, designs prioritizing far vision and designs prioritizing near vision, etc., a lens design that results in the behavioral pattern during gaze shifting that is more desirable as intended by a designer (for example, shorter reaction time and greater postural stability) is selected, to thereby suggest a design that is more suited to the subject.

In this example, the design of the progressive power lens suited to the subject was selected based on the data on the average reaction time and postural stability, but the design can also be selected based on, for example, the percentage of correct subjective reactions or the variation (symmetry) of the reaction times.

### (2) Regarding asymmetry of the reaction time

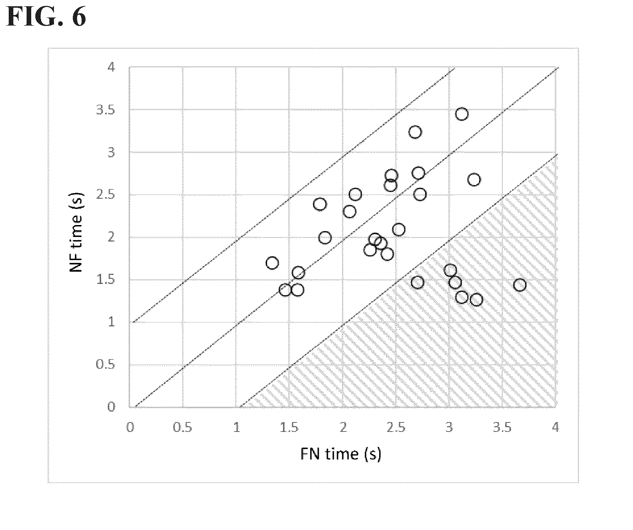

In addition, data on the behavioral pattern (asymmetry of the reaction time) during each subject's gaze shifting measured by the above gaze shifting task was plotted and organized in FIG. 6. In FIG. 6, the horizontal axis indicates the average reaction time when switching from far vision to near vision (FN time), and the vertical axis indicates the average reaction time when switching from near vision to far vision (NF time). Usually, there is no significant difference in the time it takes for each gaze shifting, but asymmetry in the reaction time may appear in some cases. In these cases, the subjects were judged that there was room for more comfortable use of the progressive power lens.

The subject in the shaded area in the lower right of FIG. 6 took longer to shift his or her gaze from far vision to near vision than it did to shift his or her gaze from near vision to far vision. Therefore, it is considered that a gaze passing position for near vision or a gaze passing position in the intermediate portion is not stable, or a wearer is not familiar with using his or her gaze when wearing the progressive power lens, and so it takes time to refocus. For such subjects, a design that expands the stable power range in the left and right directions for near vision, was selected. This is considered to facilitate refocusing because a required adjustment power does not change even when the gaze is shifted slightly left or right.

As described above, it was confirmed that by the gaze shifting task, the subject's behavioral pattern during gaze shifting was measured, and that the design of the progressive power lens that is most suited to the subject can be selected (determined) based on the data on the measured behavioral pattern during gaze shifting.

### Description of signs and numerals

10 Subject
20 First display unit
21 Second display unit
30 Optotype
40 Input unit
50 Imaging unit
S101 Optotype display step
S102 Subjective reaction acquisition step
S103 Design selection step

## Claims

1. A method for measuring a behavioral pattern during gaze shifting, the method comprising: displaying an optotype on at least one of multiple display units arranged at different distances from a subject wearing a progressive addition lens, and allowing the subject to view the optotype; and obtaining a subjective reaction from the subject who views the optotype, wherein by repeating the viewing a plurality of optotypes and obtaining the subjective reaction, the subject is allowed to experience two or more different viewing states and his or her behavior is measured between the different viewing states.

2. The method for measuring a behavioral pattern during gaze shifting according to claim 1, wherein the change in behavior includes a change in at least one of the following: whether the subjective reaction is correct or incorrect; the reaction time from when the optotype is displayed until the subjective reaction is obtained; and a movement of a subject's head or eyeballs from when the optotype is displayed until the subjective reaction is obtained.

3. The method for measuring a behavioral pattern during gaze shifting according to claim 1, wherein the two or more different viewing states include a near vision state and a far vision state.

4. The method for measuring a behavioral pattern during gaze shifting according to claim 1, wherein when repeating the viewing the optotype and the obtaining the subjective reaction, the optotype is displayed in a certain order so that the subject performs a series of gaze shifting at a discrete viewing distance.

5. The method for measuring a behavioral pattern during gaze shifting according to claim 1, wherein in the viewing the optotype and the obtaining the subjective reaction, the subject is continuously photographed, and a movement of a subject's head or eyeballs is measured from when the optotype is displayed until when the subjective reaction is obtained.

6. A method for determining a progressive power lens, the method comprising:
displaying an optotype on at least one of multiple display units arranged at different distances from a subject wearing a progressive power lens, and allowing the subject to view the optotype; and
obtaining a subjective reaction from the subject who views the optotype,
the method further comprising:
allowing the subject to experience two or more different viewing states by repeating the viewing of the optotype and the obtaining the subjective reaction for multiple optotypes, and measuring a change in subject's behavior between the viewing states; and
selecting a design of the progressive power lens suited to the subject based on data on the change in behavior.

7. A device for measuring a behavioral pattern during gaze shifting, the device comprising:
multiple display units arranged at different distances from a subject wearing a progressive power lens, for displaying optotypes to be viewed by the subject;
an input unit into which the subject who views the optotype inputs a subjective reaction; and
a measurement unit that allows the subject to experience two or more different viewing states by repeating the display of the optotypes and the input of the subjective reaction, for measuring a change in subject's behavior between the viewing states.

8. The device for measuring a behavioral pattern during gaze shifting according to claim 7, the device further comprising an imaging unit that continuously images the subject.
